# FASCICULE DE BREVET EUROPEEN

(11) **EP 2 331 151 B1**
(45) Date de publication et mention de la délivrance du brevet: **11.07.2012**
(21) Numéro de dépôt: 09782113.6
(22) Date de dépôt: 24.08.2009
(51) Int. Cl.: A61L 27/02, A61L 27/10

(54) **SUBSTITUT OSSEUX À BASE DE BIOVERRE POREUX ET DE SULFATE DE CALCIUM**
PORÖSES BIOGLAS UND CALCIUMSULFAT ENTHALTENDER KNOCHENERSATZ
BONE SUBSTITUTE CONTAINING POROUS BIO-GLASS AND CALCIUM SULPHATE

(30) Priorité: 27.08.2008 BE 200800474
(43) Date de publication de la demande: 15.06.2011
(73) Titulaire: Noraker, 69100 Villeurbanne (FR)
(72) Inventeur: RAUIS, André, 1050 Bruxelles (BE); ZENATI, Rachid, 69250 Neuville sur Saone (FR)
(74) Mandataire: Brédeville, Odile Marie
(86) Numéro de dépôt international: PCT/EP2009/060874
(87) Numéro de publication internationale: WO 2010/023179

(56) Documents cités:
- WO-A1-03/011957
- WO-A1-2008/077257
- US-A1- 2003 055 512
- CAMARGO ET AL: "Influence of bioactive glass on changes in alveolar process dimensions after exodontia" ORAL SURGERY ORAL MEDICINE ORAL PATHOLOGY, vol. 90, no. 5, 1 novembre 2000 (2000-11-01), pages 581-586, XP002584645

## Description

La présente invention concerne une composition chimique nouvelle de substitut osseux, composée d'un mélange de bioverre poreux et de sulfate de calcium.

Les défects osseux rencontrés en chirurgie orthopédique et en art dentaire sont depuis longtemps traités par greffe osseuse. La greffe est prélevée soit chez le patient (auto-greffe), ce qui implique un acte chirurgical supplémentaire, soit obtenue auprès d'une banque de tissus humains (allogreffe) ce qui implique des coûts plus élevés et des risques de contamination. Aussi, depuis longtemps, la chirurgie tente de faire appel à des substituts osseux de nature chimique.

Depuis plus d'un siècle, le sulfate de calcium (« plâtre de Paris ») a été utilisé dans le but de combler les défects osseux (comblement de cavités d'ostéïte, de carries vertébrales dans la tuberculose osseuse, etc...) avec des résultats appréciables. Il apporte le matériau (Ca++) et la trame (« scaffold ») nécessaires à la repousse osseuse mais, pêche cependant par deux défauts : il est trop rapidement résorbé, et ne stimule en rien la croissance osseuse.

D'autres subtituts chimiques sont actuellement utilisés : par exemple :
- L' HAP (hydroxyapatite)
- L'HAP biphasique
- Le carbonate d'HAP
- Le beta TCP (calcium phosphate biphasique)
- DCTD (dicalcium phosphate dihydrate)
- L'HAP + silicium
- L'HAP + collagène
- Le sulfate de calcium
- Le sulfate de calcium + PLLA
- Le bioverre (« bioglass »®)
- Le bioverre poreux
- et des associations éventuelles de ces produits.

Certains de ces produits et composés chimiques ont le défaut d'être très lentement résorbables par l'organisme et s'opposent donc par leur présence à la repousse osseuse (HAP, betaTCP et DCPD)

Le bioverre est résorbable. Le temps de résorption étant fonction inverse de la surface de contact. Ainsi le bioverre poreux présentant une importante surface de contact avec les fluides de l'organisme aura une résorption rapide à l'inverse du bioverre brut.

Parmi les 47 substituts osseux commercialisés relevés par l'AAOS (American Academy of Orthopedic Surgeons, Janv. 2008), aucun ne correspond au substitut osseux idéal.

D'autres articles de référence dans ce domaine sont : Kelly, C.M. et al, « The use of a surgical Grade Calcium Sulfate as a Bone Graft Substitue », Clin. Orthop. Rel. Res. 382, 2001, pp 42-50 Peltier, L. F., "The Use of Plaster of Paris to Fill Defects in Bone", Clin.Orthop. Rel. Res.21, 1961, pp. 1-31

Alexander, D. et al, "Efficacy of Calcium Sulfate Plus Decompression Bone in Lumbar and Lumbosacral Spinal Fusion : Preliminary Results in 40 patients", Can. J. Surg., 44(4), 2001, pp. 262-266. Schepers et al., "Bioactive Glass Particles on Narrow Size Range : A New Material for the Repair of Bone Defects", Implant Dent. 1993, 2, 151-156.

On peut ainsi admettre que les qualités requises d'un substitut idéal sont :
1. apporter le matériau osseux (CA++)
2. apporter une trame (« scaffold ») permettant une ostéoconduction, c'est-à-dire un support pour la repousse osseuse.
3. être résorbable en un laps de temps convenable afin de ne pas s'opposer par sa présence à la repousse osseuse.
4. apporter une ostéoinduction, c'est-à-dire une stimulation de la repousse osseuse en favorisant le développement des cellules osseuses.
5. être, pendant la phase de préparation, malléable et même injectable à l'aide d'une seringue ou d'un trocard.
6. apporter une prise mécanique rapide avec une résistance mécanique à la compression analogue à celle de l'os spongieux.

Le document WO 2008/077257 décrit un substitut osseux composé d'un mélange de précurseur d'hydrogel et de particules poreuses solides, tel qu'un mélange de polymorphes de sulfate de calcium hémihydrate et de bioverre, sous forme de particules ayant un diamètre entre 100 et 500 micromètres, une porosité entre 60 et 90% et une dimension de pores entre 100 nanomètres et 500 micromètres. La composition est rendue injectable par l'ajout d'eau ou de solution saline.

Le document US 2003/055512 divulgue des compositions injectables pour ciments osseux, comprenant du sulfate de calcium et des phosphates de calcium en poudre en combinaison avec de l'eau ou une solution de NaCl.

Il s'est avéré que ces substituts osseux connus ne possèdent pas l'ensemble des qualités requises pour un substitut osseux idéal, évoqué ci-dessus.

Parmi les produits chimiques convenant pour une implantation dans l'organisme d'un mammifère (ea l'homme) on peut citer :
1. Le bioverre (SiO2, CaO, Na2O, P2O5) ou (« Bioglass 45S5 »®):
   - Ostéostimulant
   - Apportant les matériaux calcium et phosphore
   - Mais à résorption lente, et non malléable.
2. Le bioverre poreux (tel que défini dans les documents WO 2006/018531 A2 et US 2008/0038534 A1 de l'INSA) ou tel que défini dans les documents US 2007/0162151 A1, US 5 648 301 A, US 5 676 720 B1, US 6 406 498 A et US6413538 B1)
   - Par la taille de ses pores il offre une ostéoconduction pour la repousse des cellules osseuses.
   - Il est puissamment ostéoinducteur.
   - Sa grande surface de contact avec les fluides de l'organisme lui confère une résorption plus rapide que le bioverre compact.

Le sulfate de calcium (CaSO4, « plâtre de Paris ») de qualité médicale est utilisé depuis plus d'un siècle comme substitut osseux pour combler des défects osseux avec des résultats appréciables. Il apporte le calcium nécessaire à la reconstruction de l'os, il est ostéoconducteur, il est malléable, injectable, offre une prise mécanique rapide, et se résorbe rapidement (en 4 à 8 semaines).

Il est actuellement largement utilisé et commercialisé comme substitut osseux sous les noms de
- Surgiplaster (Orthogen Corporation)
- Calceon 6 (Synthes USA)
- Ceraplast (Ceraver)
- Surgical plaster (ACE CO)
- Ostéoset (Wright Medical Technologies)
- MIIG 115 (Wright Medical Technologies)
- MIIG x 3 (Wright Medical Technologies),
   dont certains font l'objet des brevets :
- US 5614 206
- US 5807 567
- US 6030 636
- US 6652 887

Les deux produits chimiques que sont le bioverre et le sulfate de calcium paraitraient a priori complémentaires pour que leur mélange aboutisse à un substitut osseux idéal comme défini plus haut.

Cependant le mélange et la préparation de granulés de bioverre bruts et de plâtre s'avèrent non appropriés car, en présence de bioverre brut, la prise du CaSO4 ne se réalise pas.

Il a par contre été constaté que le bioverre poreux tel que défini dans le document WO 2006/018531 A2, obtenu à partir du bioverre brut peut, sous certaines conditions sous forme de granulés, réaliser avec le sulfate de calcium, un mélange qui est injectable et solidifie rapidement offrant alors une excellente résistance mécanique.

La présente invention concerne une telle composition de granulés de bioverre poreux et de sulfate de calcium, et plus particulièrement une composition pulvérulente pour substitut osseux comprenant du bioverre poreux en poudre ou en granules et du sulfate de calcium alfa hémihydrate en poudre.

Selon l'invention la composition comprend de 0,25 à 5 parts en poids de sulfate de calcium alfa hémihydrate pour 1 part de bioverre.

Selon une autre particularité préférée de l'invention la granulométrie du bioverre poreux est comprise entre 0 et 1000 microns (plus particulièrement entre 1 et 1000 microns).

Le premier matériau, le bioverre poreux, (fourni, par exemple, par la firme « Noraker ») peut se présenter sous la forme de granulés de taille variable, à savoir de :
- Granulométrie de 1 à 90 µ.
- Granulométrie de 90 à 200 µ.
- Granulométrie de 200 à 300 µ.
- Granulométrie de 300 à 900 µ.
- et autres dimensions désirables

Selon l'invention, la taille des granulés de bioverre poreux a une grande importance.

En effet des granules de 1 à 90 µ montrent une prise trop lente du mélange. D'autre part, des granulés de 900 µ montrent une bonne prise du mélange mais au détriment de l'injectabilité du fait de leur taille trop importante.

Selon une caractéristique préférée de l'invention, la taille idéale se situe entre 100 et 300 p.

Le deuxième matériau, le sulfate de calcium hémihydrate, se présente sous forme de poudre fine qui, mélangée à de l'eau, donne par réaction chimique le sulfate de calcium dihydrate, particulièrement résistant mécaniquement.

Le sulfate de calcium hémihydrate existe sous deux formes cristallines : alpha hémihydrate et béta hémihydrate. La forme alpha donnera après mélange avec l'eau un dihydrate plus solide que le béta hémihydrate et aura, in vivo, une résorption plus lente (de l'ordre de 30 jours pour béta et de 50 jours pour l'alpha).

Il est donc impératif de choisir, pour la composition selon l'invention, un hémihydrate de type alpha.

Celui-ci peut être obtenu à partir d'un procédé particulier de chauffage de dihydrate, soit par procédé chimique (déhydratation du dihydrate par chauffage en solution diluée d'acide sulfurique).

Une poudre de alpha hémihydrate est notamment commercialisée sous le nom de CAPSET® de Lifecore, Chaska, Minn.

Pour que la prise solide se réalise en un temps bref, il est apparu hautement préférable d'adjoindre un accélérateur de prise. Selon une caractéristique préférée de l'invention la composition comprend dès lors au moins un accélérateur de prise choisi parmi :
- le NaCl
- le dihydrate finement moulu (taille de particules inf. à 50µ) à une dose variant entre 1 et 15% du poids d'hémihydrate. En milieu protéïque, la prise du mélange requiert en tous cas la présence de dihydrate.

Le sulfate de calcium dihydrate a de préférence une granulaométrie comprise 10 et 100 microns.

La firme Lifecore, Chaska, Minn. fournit un alpha hémihydrate préchargé de dihydrate sous le nom de HAPSET®.

La composition pulvérulente selon l'invention comprend de préférence 1 à 15% en poids de sulfate de calcium dihydrate par rapport au sulfate de calcium alfa hémihydrate, et plus particulièrement
de 20 à 80% en poids du mélange de bioverre poreux en poudre ou en granules,
de 20 à 80% en poids du mélange de sulfate de calcium alfa hémihydrate,
de 1 à 15% en poids du mélange de sulfate de calcium dihydrate, avec, optionnellement de 0 à 2% en poids du mélange de NaCl.

Le bioverre poreux utilisé a de préférence une porosité (mesurée par méthode géométrique) de 50 à 80%, avec des macropores ayant un diamètre moyen de 100 à 1250 microns et des micropores ayant un diamètre moyen infrieur ou égal à 5 microns, et a de préférence une teneur en SiO₂ de 40 à 55% en poids, en CaO de 15 à 25% en poids, en Na₂O de 15 à 25% en poids, en P₂O₅ de 1 à 9% en poids.

Une composition pulvérulente préférée selon l'invention comprend :
1. du bioverre poreux en granulés (de 10 à 900µ), dans une proportion de 20 à 80% en poids du mélange
2. du sulfate de calcium alpha hémihydrate en poudre dans une proportion de 20 à 80% en poids du mélange,
3. du sulfate de calcium dihydrate (en poudre fine), dans une proportion de 1 à 15% en poids du mélange,
4. éventuellement du NaCl, dans une proportion de 0 à 2% en poids du mélange
5. et éventuellement de la hydroxy propyl méthyl cellulose, dans une proportion de 0 à 2% en poids du mélange.

La préparation peut se faire de la façon suivante : les poudres de CaSO4 alphahémihydrate et CaSO4 dihydrate et les granulés de bioverre poreux sont mélangés à sec dans un récipient à température ambiante. Le mélange est éventuellement additionné de NaCl et/ou de hydroxy propyl méthyl cellulose.

L'invention concerne également une composition pour substitut osseux injectable, comprenant une composition pulvérulente telle que décrite plus haut, mélangée avec de l'eau déionisée, à température ambiante. La préparation est de préférence mélangée pendant trente secondes.

Une composition préférée pour substitut osseux injectable, selon l'invention, comprend :
1. du bioverre poreux en granulés (de 10 à 900µ), dans une proportion de 20 à 80% en poids du mélange
2. du sulfate de calcium alpha hémihydrate en poudre dans une proportion de 20 à 80% en poids du mélange,
3. du sulfate de calcium dihydrate (en poudre fine), dans une proportion de 1 à 15% en poids du mélange,
4. de l'eau déïonisée en quantité convenable
   (de préférence de 10 à 50 % en poids du mélange sec)
5. éventuellement du NaCl, dans une proportion de 0 à 2% en poids du mélange
6. et éventuellement de la hydroxy propyl méthyl cellulose, dans une proportion de 0 à 2% en poids du mélange.

La prise du mélange CaSO4 alphahémihydrate, CaSO4 dihydrate, granulés de bioverre poreux et eau déöinisée, additionné ou non NaCl et/ou de hydroxy propyl méthyl cellulose, se fait en trois phases :
- Après mélange des composants, la composition devient plastique et injectable, ceci pendant quelques minutes.
- Puis apparaît le « moment de prise initiale » (« Initial Setting Time » = IST) :
   La composition n'est plus malléable ni injectable mais ne présente pas encore
   de prise à la compression.
- Un laps de temps supplémentaire (de 5 à 15 min.) amène à une prise finale (« Final Setting Time » = FST) présentant une résistance à la compression de l'ordre de 1 MPa), analogue à la résistance de l'os spongieux porotique. Après une heure la résistance atteint 8 MPa.

L'adjonction éventuelle hydroxy propyl méthyl cellulose agit comme plastifiant qui peut améliorer l'injectabilité du mélange. Le NaCl peut être utilisé comme accélérateur supplémentaire de prise.

Les compositions pour substitut osseux, selon l'invention, sont injectables, ostéoconductrices, ostéinductrices, résorbables et offrent une résistance mécanique proche de celle de l'os.

### Exemple 1 :

Une composition pour substitut osseux injectable, selon l'invention, peut être réalisé avec :
- 10 grammes de bioverre poreux en granulés de 100 à 300µ.
- 10 grammes de sulfate de calcium alpha hémihydrate.
- 1 gramme de dihydrate poudre fine.
- 12 grammes d'eau désïonisée.

Les trois premiers matériaux sont mélangés à sec puis additionnés de l'eau et mélangés pendant 30 secondes.

L'injectabilité est possible pendant 4 minutes (jusqu'au temps de prise IST). La prise solide se fait en 10 minutes (FST).

### Exemple 2 :

Une autre composition pour substitut osseux injectable, selon l'invention, peut être réalisé avec :
- 10 grammes de bioverre poreux en granulés de 100 à 300µ.
- 20 grammes de sulfate de calcium alpha hémihydrate.
- 2 grammes de sulfate de calcium dihydrate.
- 0,1 gramme de NaCl.

L'injectabilité est possible pendant 6 minutes et la prise solide après 17 minutes.

### Exemple 3 :

Une composition pour substitut osseux injectable, selon l'invention, peut être réalisé comme dans l'exemple 2, en y ajouttant 1 gramme d'hydroxy propyl méthyl cellulose.

La viscosité des mélanges selon les examples 1 - 3, pendant la phase de préparation avant la prise initiale (IST), est telle qu'elle permet de l'injecter à l'aide d'une seringue ou de le manipuler comme un mastic de comblement.

La faible réaction exothermique lors de la prise du mélange (inférieure à 40°C) autorise l'incorporation au mélange d'agents médicamenteux. Par exemple, des antibiotiques
(Gentamycine, Tobramycine, Cephalosporines, Vencomycine,...), des agents de chimiothérapie (Cis-platinum, Méthotrexate, Isofosfamide, ...), des analgésiques (Lidocaïne, ...)

Les deux composants principaux de la composition auront, après implantation dans l'organisme d'un mammifère (e.a. l'homme), des vitesses de résorption différentes.

Le sulfate de calcium se résorbera le premier pour avoir disparu en environ cinquante jours, et cette résorption laissera progressivement apparaître au contact des tissus le bioverre poreux dont la résorption est plus lente permettant à celui-ci d'apporter son action d'ostéostimulation. Ainsi la résorption graduée des deux composants se fait à un rythme correspondant à la vitesse de repousse osseuse.

## Revendications

1. Composition pulvérulente pour substitut osseux à base de bioverre poreux, **caractérisée en ce que** la composition comprend du bioverre poreux en poudre ou en granules et de 0,25 à 5 parts de sulfate de calcium alfa hémihydrate en poudre pour 1 part de bioverre.

2. Composition selon la revendication 1, **caractérisée en ce que** la granulométrie du bioverre poreux est comprise entre 0 et 1000 microns.

3. Composition selon l'une ou l'autre des revendications précédentes, **caractérisée en ce que** la composition comprend au moins un accélérateur de prise choisi parmi le sulfate de calcium dihydrate finement moulu et le chlorure de sodium.

4. Composition selon la revendication 3, **caractérisée en ce que** le sulfate de calcium dihydrate a une granulaométrie comprise 10 et 100 microns.

5. Composition selon l'une ou l'autre des revendications 3 et 4, **caractérisée en ce que** la composition comprend 1 à 15% en poids de sulfate de calcium dihydrate par rapport au sulfate de calcium alfa hémihydrate.

6. Composition selon l'une ou l'autre des revendications 3 à 5, **caractérisée en ce que** la composition comprend
de 20 à 80% en poids du mélange de bioverre poreux en poudre ou en granules,
de 20 à 80% en poids du mélange de sulfate de calcium alfa hémihydrate, de 1 à 15% en poids du mélange de sulfate de calcium dihydrate, et, optionnellement de 0 à 2% en poids du mélange de NaCl.

7. Composition selon l'une ou l'autre des revendications précédentes, **caractérisée en ce que** le bioverre poreux a une porosité (mesurée par méthode géométrique) de 50 à 80%, avec des macropores ayant un diamètre moyen de 100 à 1250 microns et des micropores ayant un diamètre moyen inférieur ou égal à 5 microns.

8. Composition selon l'une ou l'autre des revendications précédentes, **caractérisée en ce que** le bioverre poreux a une teneur en SiO₂ de 40 à 55% en poids, en CaO de 15 à 25% en poids, en Na₂O de 15 à 25% en poids, en P₂O₅ de 1 à 9% en poids.

9. Composition pour substitut osseux injectable, comprenant un mélange d'eau et d'une composition pulvérulente selon l'une ou l'autre des revendications précédentes.

10. Composition selon la revendication 9, **caractérisée en ce qu'**elle comprend de 10 à 50 % en poids d'eau par rapport à la quantité de matière sèche.

11. Composition selon l'une ou l'autre des revendications 9 - 10, **caractérisée en ce qu'**elle comprend de 0,1 à 2% en poids de NaCl.

## Claims

1. A powdery composition for a bone substitute based on porous bioglass, **characterized in that** the composition comprises a porous bioglass in powder or in granules and from 0.25 to 5 parts of calcium sulfate alpha-hemihydrate in powder for one part of bioglass.

2. The composition according to claim 1, **characterized in that** the grain size of the porous bioglass is comprised between 0 and 1,000 microns.

3. The composition according to either one of the preceding claims, **characterized in that** the composition comprises at least one setting accelerator selected from finely milled calcium sulfate dihydrate and sodium chloride.

4. The composition according to claim 3, **characterized in that** the calcium sulfate dihydrate has a grain size comprised between 10 and 100 microns.

5. The composition according to either one of claims 3 and 4, **characterized in that** the composition comprises 1 to 15% by weight of calcium sulfate dihydrate based on the calcium sulfate alpha-hemihydrate.

6. The composition according to either one of claims 3 to 5, **characterized in that** the composition comprises
from 20 to 80% by weight of the porous bioglass mixture either in powder or granules,
from 20 to 80% by weight of the mixture of calcium sulfate alpha-hemihydrate,
from 1 to 15% by weight of the mixture of calcium sulfate dihydrate, and optionally from 0 to 2% by weight of the NaCl mixture.

7. The composition according to either one of the preceding claims, **characterized in that** the porous bioglass has a porosity (measured by a geometrical method) from 50 to 80%, with macropores having an average diameter from 100 to 1,250 microns and micropores having an average diameter of less than or equal to 5 microns.

8. The composition according to either one of the preceding claims, **characterized in that** the porous bioglass has an SiO₂ content from 40 to 55% by weight, a CaO content from 15 to 25% by weight, a Na₂O content from 15 to 25% by weight, a P₂O₅ content from 1 to 9% by weight.

9. A composition for an injectable bone substitute, comprising a mixture of water and of a powdery composition according to either one of the preceding claims.

10. The composition according to claim 9, **characterized in that** it comprises from 10 to 50% by weight of water based on the amount of dry material.

11. The composition according to either one of claims 9 to 10, **characterized in that** it comprises from 0.1 to 2% by weight of NaCl.

## Patentansprüche

1. Pulverförmige Zusammensetzung für Knochenersatz auf der Basis porösen Bioglases, **dadurch gekennzeichnet, dass** die Zusammensetzung pulverförmiges oder körniges Bioglas und 0,25 bis 5 Anteile pulverförmiges Calciumsulfat-α-Halbhydrat für einen Anteil Bioglas umfasst.

2. Zusammensetzung nach Anspruch 1, **dadurch gekennzeichnet, dass** die Korngröße des porösen Bioglases zwischen 0 und 1000 Mikron inklusive ist.

3. Zusammensetzung nach dem einen oder dem anderen der vorangehenden Ansprüche, **dadurch gekennzeichnet, dass** die Zusammensetzung mindestens einen Bindungsbeschleuniger umfasst, der aus dem fein gemahlenen Calciumsulfat-Dihydrat und dem Natriumchlorid ausgewählt ist.

4. Zusammensetzung nach Anspruch 3, **dadurch gekennzeichnet, dass** das Calciumsulfat-Dihydrat eine Korngröße von 10 und 100 Mikron inklusive hat.

5. Zusammensetzung nach dem einen oder dem anderen der Ansprüche 3 und 4, **dadurch gekennzeichnet, dass** die Zusammensetzung 1 bis 15 Gew.-% Calciumsulfat-Dihydrat im Verhältnis zum Calciumsulfat-α-Halbhydrat umfasst.

6. Zusammensetzung nach dem einen oder dem anderen der Ansprüche 3 bis 5, **dadurch gekennzeichnet, dass** die Zusammensetzung
20 bis 80 Gew.-% des pulverförmigen oder körnigen porösen Bioglasgemischs,
20 bis 80 Gew.-% des Calciumsulfat-α-Halbhydrat-Gemischs,
1 bis 15 Gew.-% des Calciumsulfat-Dihydrat-Gemischs und optional 0 bis 2 Gew.-% des NaCl-Gemischs umfasst.

7. Zusammensetzung nach dem einen oder dem anderen der vorangehenden Ansprüche, **dadurch gekennzeichnet, dass** das poröse Bioglas eine Porosität (durch geometrische Methode gemessen) von 50 bis 80 % hat mit Makroporen, die einen durchschnittlichen Durchmesser von 100 bis 1250 Mikron haben und Mikroporen, die einen durchschnittlichen Durchmesser von unter oder gleich 5 Mikron haben.

8. Zusammensetzung nach dem einen oder dem anderen der vorangehenden Ansprüche, **dadurch gekennzeichnet, dass** das poröse Bioglas einen Gehalt an SiO₂ von 40 bis 55 Gew.-%, an CaO von 15 bis 25 Gew.-%, an Na₂O von 15 bis 25 Gew.-%, an P₂O₅ von 1 bis 9 Gew.-%. hat.

9. Zusammensetzung für injizierbaren Knochenersatz, die ein Gemisch aus Wasser und einer pulverförmigen Zusammensetzung nach dem einen oder dem anderen der vorangehenden Ansprüche umfasst.

10. Zusammensetzung nach Anspruch 9, **dadurch gekennzeichnet, dass** sie 10 bis 50 Gew.-% Wasser im Verhältnis zu der Menge Trockenmaterial umfasst.

11. Zusammensetzung nach dem einen oder dem anderen der Ansprüche 9 bis 10, **dadurch gekennzeichnet, dass** sie 0,1 bis 2 Gew.-% NaC1 umfasst.
